# EUROPÄISCHE PATENTSCHRIFT

(11) **EP 0 466 012 B1**
(45) Veröffentlichungstag und Bekanntmachung des Hinweises auf die Patenterteilung: **27.09.1995**
(21) Anmeldenummer: 91111013.8
(22) Anmeldetag: 03.07.1991
(51) Int. Cl.: C01B 39/00, B01J 29/04, C07C 2/00

(54) **Verfahren zur Herstellung von kristallinen zeolithanalogen Gallosilikaten und ihre Verwendung zur Herstellung von Katalysatoren und Adsorbentien**
Method for preparation of crystalline zeolite like gallosilicates and their use for the preparation of catalysts and adsorbents
Procédé de préparation de gallosilicates cristallins analogues aux zéolites et leur utilisation pour la préparation de catalyseurs et d'adsorbants

(30) Priorität: 03.07.1990 DE 4021118
(43) Veröffentlichungstag der Anmeldung: 15.01.1992
(73) Patentinhaber: VAW Aluminium AG, D-53117 Bonn (DE); VEBA OEL AG, 45896 Gelsenkirchen (DE)
(72) Erfinder: Tissler, Arno, Dr., W-5300 Bonn 1 (DE); Thome, Roland, Dr., W-5300 Bonn 1 (DE); Wallau,Martin Dr., W-6500 Mainz 42 (DE); Spichtinger,Rudolf, W-6230 Frankfurt 80 (DE); Unger,Klaus Konradin Prof.Dr., W-6140 Bensheim 8 (DE)
(74) Vertreter: Müller-Wolff, Thomas, Dipl.-Ing.

(56) Entgegenhaltungen:
- EP-A- 0 094 288
- EP-A- 0 150 256
- EP-A- 0 323 893
- FR-A- 2 108 479
- FR-A- 2 156 098
- US-A- 4 166 099
- US-A- 4 803 060

## Beschreibung

Die Erfindung betrifft ein Verfahren zur Herstellung von kristallinen, zeolithanalogen Gallosilikaten mit Pentasilstruktur mit einem Si/Ga-Atomverhältnis von ≧ 5 durch hydrothermale Kristallisation aus einem Reaktionsansatz, der in wässrigem, alkalischem Medium SiO₂ und Ga₂O₃ bzw. deren hydratisierte Derivate oder Alkalisilikate und -gallate und ggf. quarternäre Ammoniumverbindungen enthält.

Aus der FR-A-0 094 288 ist ein Verfahren zur Herstellung von zeolithischen Materialien bekannt, bei denen auch unter anderem Gallosilikate mit ZSM-5 Struktur erhalten werden. Hierzu werden neben den üblichen Reaktionspartnern auch Wasserstofffluorosilikat eingesetzt. Derartige Reaktionsmittel haben neben ihrer Umweltproblematik auch Nachteile bei der Verwendung in Reaktionsgefäßen aus Stahl, da sie sehr korrosiv sind.

Gallosilikate mit Pentasilstruktur eignen sich besonders für den Einsatz als Katalysatoren in der Petrochemie und zur Herstellung wertvoller organischer Zwischenprodukte. Andere Zeolithstrukturen, z.B. Typ L und Faujasite-Strukturen sind in EP-A-0 327 893 und US-A-4 803 060 beschrieben.

Weiterhin sind auch Verfahren zur Herstellung zeolithanaloger Gallosilikate bekannt, die unter Verwendung von quarternären Amoniumverbindungen aus Mischungen aus reaktivem Siliziumdioxid und Gallium-III-oxid Kristalle mit stark unterschiedlicher Kristallgröße entstehen. Dabei verteuern die quarternären Amoniumverbindungen die Zeolithsynthese und verursachen beim Kalzinieren einen erheblichen Schadstoffausstoß, der zusätzliche verfahrenstechnische Maßnahmen im Rahmen des Umweltschutzes erforderlich macht.

Aufgabe der vorliegenden Erfindung ist es, bei einem Verfahren der eingangs genannten Art auf die Verwendung von strukturbildenden Schablonenverbindungen zu verzichten und zeolithanaloge Gallosilikate in Pentasilstruktur innerhalb eines engen Korngrößenbereiches vorzugsweise innerhalb von 1 bis 5 µm herzustellen.

Dieser Aufgabe wird erfindungsgemäß durch die im Anspruch 1 angegebenen Merkmale gelöst.

Überraschenderweise gelingt es durch den Zusatz von 2 bis 40 Gew.-% eines gealterten Keimgeles der molaren Zusammensetzung SiO₂/Ga₂O₃ ≧ 5; OH⁻/SiO₂ = 0,01 - 1, quarternäre Ammoniumverbindung/SiO₂ = 0-2; H₂O/SiO₂ = 10-1000 zum rein anorganischen Reaktionsansatz zeolithanaloge Gallosilikate herzustellen. Der Anteil der quarternären Ammoniumverbindung am Gesamtreaktionsvolumen kann hierbei sehr klein sein, im Einzelfall 0 betragen.

Der Anteil des im Gitter eingebauten Galliums kann bei dem erfindungsgemäßen Verfahren deutlich höher eingestellt werden (SiO₂/Ga₂O₃ ≧ 40 bei herkömmlichen Verfahren und SiO₂/Ga₂O₃ ≧ 5 beim erfindungsgemäßen Verfahren), so daß die katalytische Aktivität, die eine Funktion des im Gitter eingebauten Galliums ist, deutlich höher liegt.

Das erfindungsgemäße Verfahren zeichnet sich ferner durch seine Umweltverträglichkeit aus, da der Anteil der organischen Schablonenverbindung sehr klein, im Einzelfall 0 sein kann. Des weiteren ist das Verfahren kostengünstig und wenig energieaufwendig durchzuführen. Die notwendige Synthesetemperatur kann durch den Zusatz des Keimgeles ebenfalls niedriger gehalten werden.
Hinzu kommt, daß die erfindungsgemäßen Gallosilikate sich durch ihre einheitliche Morphologie im Vergleich zu nach dem Stand der Technik hergestellten ZAG auszeichnen.

Erfindungsgemäß erfolgt die Herstellung des Keimbildungsgels vorzugsweise aus einer Mischung von SiO₂ (z. B. 20 bis 50 g) und NaOH (z. B. 5 bis 10 g) in einer wäßrigen GaCl₃-Lösung (z. B. 5 bis 20 g). Das spezifische Gewicht der GaCl₃-Lösung beträgt 1,2 bis 1,5 g/ml, vorzugsweise 1,38 g/ml, bei einem Galliumgehalt von etwa 0,2 g/ml Wasser. Die Reaktionsmischung wird auf eine Temperatur zwischen 15 und 100 °C, vorzugsweise etwa 90 °C, aufgeheizt. Bei dieser Temperatur wird die Reaktionsmischung intensiv gerührt (100 Upm) über einen Zeitraum von 2 h bis zu 100 Tagen, vorzugsweise 7 Tage.

Optional kann dem Gel ein quarternäres Ammoniumsalz zugegeben werden. Hierzu eignen sich insbesondere Tetraalkylammoniumsalze, wie z. B. Tetrapropylammoniumbromid, Tetrapropylammoniumhydroxid, Tetramethylbromid und Tetramethylhydroxid. Die Zugabemenge an quarternären Ammoniumsalzen liegt insgesamt zwischen 0 und 270 g, wobei der Bereich zwischen 0 und 70 g vorzuziehen ist. Auf diese Weise wird das molare Verhältnis von quarternärem Ammoniumsalz zu SiO₂ zwischen 0 und 2, vorzugsweise zwischen 0 und 0,5 eingestellt.

Im Verlauf der Reaktionszeit erfolgt die Bildung des Gallosilikatgels. Das gebildete Gel wird anschließend einem Zersetzungs-bzw. Alterungsprozeß unterworfen. Dabei wird das Gel bei einer Temperatur im Bereich zwischen 15 und 100 °C, vorzugsweise zwischen 20 und 90 °C, über einen Zeitraum von 2 h bis zu 100 Tagen, vorzugsweise von 2 bis 7 Tagen, unter konstanten Bedingungen gerührt. Das auf diese Weise gealterte Gallosilikatgel weist folgende molare Verhältnisse auf:
SiO₂/Ga₂O₃ ≧ 5
OH⁻/SiO₂ = 0,05 bis 0,5
quarternäre Ammoniumverbindung/SiO₂ = 0 bis 2,0
H₂/SiO₂ = 10 bis 1000.

Bei der erfindungsgemäßen Herstellung eines kristallinen Gallosilikates wird das gealterte Gel einer wäßrigen alkalischen Lösung in einem geeigneten Reaktionsgefäß, vorzugsweise einem Autoklaven, zugegeben. Die Zugabe erfolgt unter intensivem Rühren (100 Upm) der Mischung. Die Zugabemenge an gealtertem Gel kann zwischen 2 und 40 Gew.-% der Mischung liegen, wobei Mengen zwischen 5 und 37 Gew.-% vorzuziehen sind.

Die verwendete wäßrige/alkalische Lösung weist vorzugsweise die folgende Zusammensetzung pro l Wasser auf:
0,01 bis 0,04 mol einer Ga-Verbindung (z. B. gelöst in 12 ml einer verdünnten Säurelösung)
0,1 bis 0,5 mol eines Alkali- oder Erdalkalihydroxides
0,4 bis 0,8 mol einer Si-Verbindung.

Geeignete Ga-Verbindungen sind z. B. Gallium(III)-oxid, Galliumchlorid, Galliumhydroxid, Galliumnitrat, Galliumsulfat und Alkaligallate sowie deren Mischungen. Vorzugsweise wird Gallium(III)-oxid verwendet, daß sehr leicht durch Hydrolyse von GaCl₃ gebildet werden kann. Als Si-Verbindung wird vorzugsweise SiO₂ eingesetzt. Weitere mögliche Si-Verbindungen sind Siliziumhydroxid, Alkalisilikate, Si(OAc)₄ und Na-Wasserglas sowie deren Mischungen. Zu den geeigneten Säurelösungen zählen insbesondere anorganische Säuren, wie z. B. Salzsäure, Schwefelsäure und Phosphorsäure. Die molare Konzentration der Säurelösung sollte 0,01 bis 5, vorzugsweise 3, betragen. Als Metallhydroxid wird vorzugsweise NaOH verwendet. Ebenfalls geeignet sind hier KOH, Ca(OH)₂ und LiOH · H₂O.

Die auf diese Weise hergestellte Reaktionsmischung weist folgende molare Zusammensetzung auf:
SiO₂/Ga₂O₃ ≧ 5
OH⁻/SiO₂ = 0,05 bis 0,5
quarternäre Ammoniumverbindung/SiO₂ = 0 bis 0,5
H₂O/SiO₂ = 20 bis 100
Alkali- bzw. Erdalkalimetall/SiO₂ = 0,3 bis 3.

Optional kann der Reaktionsmischung ein Fluoridsalz zur Stabilisierung der Synthesebedingungen zugegeben werden. Als besonders geeignet haben sich Natriumfluorid und Ammoniumfluorid erwiesen. Die Zugabemenge an Fluorid wird in diesem Falle so gewählt, daß das molare Verhältnis von Fluorid/SiO₂ in der Reaktionsmischung auf einen Wert zwischen 0,4 und 1,5, vorzugsweise zwischen 0,6 und 1, eingestellt wird.

Ebenfalls optional kann der Reaktionsmischung ein teilweise oder vollständig kristallines Gallosilikat als Impfmaterial zugegeben werden, um die Kristallisationsgeschwindigkeit zu erhöhen. Die Zugabemenge kann in diesem Fall zwischen 0,1 und 50 Gew.-%, vorzugsweise zwischen 1 und 10 Gew.-%, bezogen auf die Gesamtmischung liegen.

Die Temperatur der Reaktionsmischung wird bei der Zugabe des Gels in die alkalische Lösung auf einen Wert zwischen 25 und 100 °C, vorzugsweise auf etwa 90 °C, eingestellt. Nachdem die Zugabe des gealterten Keimbildungsgels vollständig abgeschlossen ist, erfolgt die Kristallisation unter autogenem Druck. Während der Kristallisation wird die Temperatur konstant gehalten auf einen Wert zwischen 40 und 300 °C, vorzugsweise zwischen 100 und 200 °C. Die Kristallisationsdauer liegt zwischen 1 und 72 h, vorzugsweise zwischen 24 und 48 h.

Der Kristallisation des Gallosilikates nach dem erfindungsgemäßen Verfahren kann entweder in einer einzigen Verfahrensstufe bei konstanter Temperatur und einer vorgegebenen Dauer oder in mehreren aufeinanderfolgenden Stufen erfolgen. Im letztgenannten Fall wird die Reaktionsmischung in unterschiedlichen Zeitintervallen bei unterschiedlichen konstanten Temperaturen behandelt, wobei sich diese Temperaturen und Zeiten innerhalb der vorgenannten Bereiche bewegen.

Nach Ablauf der Kristallisationszeit wird das kristalline Gallosilikat auf konventionelle Weise, z. B. durch Filtration, aus der Mischung entfernt. Anhaftende Verunreinigungen werden mit Wasser abgewaschen. Anschließend erfolgt eine Trocknung bei etwa 110 °C über einen Zeitraum von etwa 12 h. Die durchschnittliche Ausbeute an Gallosilikatkristallen liegt unter den beschriebenen Bedingungen zwischen 20 und 40 g.

Das nach dem erfindungsgemäßen Verfahren hergestellte kristalline Gallosilikat weist ein molares Verhältnis von SiO₂/Ga₂O₃ von 5 bis 100 auf. Die Partikelgröße des Gallosilikates liegt zwischen 3 und 15 µm, durchschnittlich bei etwa 10 µm. Die katalytische Aktivität der Gallosilikatkristalle, gemessen an der Umwandlung von Propan zu Aromaten bei einer Temperatur von 500 °C, einer Reaktorbelastung von 2 h⁻¹ und Normaldruck, liegt zwischen 6 und 25 %, durchschnittlich bei 15 %.

Im folgenden wird das erfindungsgemäße Verfahren anhand von Ausführungsbeispielen näher erläutert.

### Beispiel 1 (Vergleichsbeispiel)

Herstellung eines Gallosilikaten nach herkömmlicher Weise mit Hilfe einer Schablonenverbindung.
Ein homogenisierter Reaktionsansatz bestehend aus
100 g Wasser
6,07 g SiO₂
0,112 g Gallium (gel. in verd. HCl)
1,476 g NaOH
7,574 g TPABr (Tetrapropylammoniumbromid)
und den molaren Verhältnissen
H₂O/SiO₂ = 55
SiO₂/Ga₂O₃ = 125
OH⁻/SiO₂ = 0,07
TPA⁺/SiO₂ = 0,282
wurde 96 h bei 160 °C in einem Autoklaven unter autogenem Druck umgesetzt.
Man erhält nach Filtrierung und Waschung und Trocknung bei 110 °C/12 h 4,5 g eines vollkristallinen Gallosilikates mit der chemischen Zusammensetzung 124SiO₂ · Ga₂O₃ · 1,1Na₂O, das im Röntgendiffraktogramm zumindest die den in Tabelle 1 aufgelisteten Netzebenenabständen zugehörigen Röntgenreflexe aufweist. Die Kristallitgröße der Primärkristalle schwankt zwischen 0,1 - 10 µm. Diese sind zu Agglomeraten von 5 - 20 µm zusammengewachsen. Die katalytische Aktivität, gemessen als Umsatz bei der Propanumwandlung zu Aromaten bei einer Temperatur von 500 °C, einer Reaktorbelastung von 2 h⁻¹, bei Normaldruck liegt bei 4 %.

### Beispiel 2

- 36,687 g Keimgel bestehend aus:: 35,0 g H₂O
2,918 g SiO₂
0,057 g Ga₂O₃
0,385 g NaOH
0,375 g GaCl₃-Lösung

(Die GaCl₃-Lösung hat eine Dichte von 1,38 g/ml und einen Ga-Gehalt von 0,2 g Ga/ml.) werden 7 Tage bei 90 °C gealtert.
Dieses Gel wird mit
4,997 g Gallium gelöst in verd. HCl
11,351 g NaOH
1000 g Wasser
40,593 g SiO₂ (RW-Füller)
in einem Autoklaven gemischt und homogenisiert. Der Reaktionsansatz mit den molaren Verhältnissen
H₂O/SiO₂ = 83
SiO₂/Ga₂O₃ = 126
OH⁻/SiO₂ = 0,375
wird - unter autogenem Druck - sechs Stunden bei 140 °C, weitere sechs Stunden bei 165 °C und dann 1,5 Tage hydrothermal bei 180 °C umgesetzt. Man erhält nach Filtration und Waschung und Trocknung bei 110 °C/12 h ca. 50 g eines vollständig kristallinen Gallosilikates mit der chemischen Zusammensetzung 98SiO₂ · Ga₂O₃ · 1,1Na₂O, das im Röntgendiffraktogramm zumindest die den in Tabelle 1 aufgelisteten Netzebenenabständen zugehörigen Röntgenreflexe aufweist.
Die KristallitgröBe liegt zwischen 3 - 5 µm.
Die katalytische Aktivität, gemessen als Umsatz der Propanumwandlung zu Aromaten, bei einer Temperatur von 500 °C, einer Reaktorbelastung von 2 h⁻¹ bei Normaldruck liegt bei 6 %.

### Beispiel 3

40,210 g eines sieben Tage bei 90 °C gealterten Keimgeles mit der folgenden molaren Zusammensetzung:
H₂O/SiO₂ = 55
SiO₂/Ga₂O₃ = 60
OH⁻/SiO₂ = 0,07
TPA⁺/SiO₂ = 0,14
wird zu einem homogenisierten Reaktionsansatz bestehend aus
65 g Wasser
2,047 g SiO₂
0,079 g Gallium (gelöst in verd. HCl) und
1,827 g NaOH
gegeben.
Dieser Reaktionsansatz mit den molaren Verhältnissen
H₂O/SiO₂ = 80
SiO₂/Ga₂O₃ = 60
OH⁻/SiO₂ = 0,45
TPA⁺/SiO₂ = 0,07
wird 48 Stunden bei 180 °C unter autogenem Druck umgesetzt. Man erhält nach Filtration und Waschung und Trocknung bei 110 °C/12 h ca. 4,5 g eines vollständig kristallinen Gallosilikates mit der chemischen Zusammensetzung 51SiO₂ · Ga₂O₃ · 1,1Na₂O, das im Röntgendiffraktogramm zumindest die den in Tabelle 1 aufgelisteten Netzebenenabständen zugehörigen Röntgenreflexe aufweist.
Die Kristallitgröße liegt zwischen 1 - 3 µm.
Die katalytische Aktivität, gemessen als Umsatz bei der Propanumwandlung zu Aromaten bei einer Temperatur von 500 °C, einer Reaktorbelastung von 2 h⁻¹ bei Normaldruck liegt bei 15 %.

### Beispiel 4

9,9960 g eines drei Tage bei 90 °C gealterten Keimgeles mit der folgenden molaren Zusammensetzung:
H₂O/SiO₂ = 55
SiO₂/Ga₂O₃ = 50
OH⁻/SiO₂ = 0,07
TPA⁺/SiO₂ = 0,14
wird zu einem homogenisierten Reaktionsansatz bestehend aus 65 g Wasser
2,047 g SiO₂
1,1 g Gallium-Lösung
1,424 g Natriumhydroxid
gegeben.
Dieser Reaktionsansatz mit den molaren Verhältnissen
H₂O/SiO₂ = 80
SiO₂/Ga₂O₃ = 30
OH⁻/SiO₂ = 0,45
TPA⁺/SiO₂ = 0,09
wird 25 Stunden bei 180 °C unter autogenem Druck umgesetzt. Man erhält nach Filtration, Waschung und Trocknung bei 110 °C/12 h ein vollständig kristallines Gallosilikat mit der chemischen Zusammensetzung 26SiO₂ · Ga₂O₃ · 1,1Na₂O, das im Röntgendiffraktogramm zumindest die den in Tabelle 1 aufgelisteten Netzebenenabständen zugehörigen Röntgenreflexe aufweist.
Die Kristallitgröße liegt bei 1 µm.
Die katalytische Aktivität, gemessen als Umsatz bei der Propanumwandlung zu Aromaten bei einer Temperatur von 500 °C, einer Reaktorbelastung von 2 h⁻¹ bei Normaldruck liegt bei 25 %.

**Tabelle 1**

| Netzebenenabstände "d" der in den genannten Beispielen 1 bis 4 synthetisierten Gallosilikate | |
|---|---|
| d [Angström] | Intensität [-] |
| 11,2 ± 0,2 | stark |
| 10,0 ± 0,2 | stark |
| 6,4 ± 0,1 | schwach |
| 5,95 ± 0,1 | schwach |
| 5,6 ± 0,1 | schwach |
| 3,87 ± 0,05 | stark |
| 3,83 ± 0,05 | stark |
| 3,76 ± 0,05 | schwach |
| 3,74 ± 0,05 | mittelschwach |
| 3,66 ± 0,05 | schwach |
| 2,01 ± 0,02 | schwach |
| 1,99 ± 0,02 | schwach |

## Patentansprüche

1. Verfahren zur Herstellung von kristallinen, zeolithanalogen Gallosilikaten mit Pentasilstruktur mit einem Si/Ga-Atomverhältnis von ≧ 5 durch hydrothermale Kristallisation aus einem Reaktionsansatz, der in wässrigem, alkalischem Medium SiO₂ und Ga₂O₃ bzw. deren hydratisierte Derivate oder Alkalisilikate und -gallate und ggf. quarternäre Ammoniumverbindungen enthält,
dadurch gekennzeichnet,
daß man dem Reaktionsansatz als Kristallisationsbeschleuniger ein gealtertes, aber noch röntgenamorphes Gallosilikatkeimbildungsgel mit einem Si/Ga-Atomverhältnis von ≧ 5 zusetzt.

2. Verfahren nach Anspruch 1, dadurch gekennzeichnet, daß man ein gealtertes Keimbildungsgel der Zusammensetzung (auf molarer Basis)
SiO₂/GaO₂ ≧ 5
OH⁻/SiO₂⁻ = 0,01 bis 1,0
quarternärer Ammoniumverbindung/SiO₂ = 0,0 - 2,0
H₂O/SiO₂ = 10 - 1000
verwendet.

3. Verfahren nach einem der vorhergehenden Ansprüche, dadurch gekennzeichnet, daß im Keimbildungsgel folgende molare Verhältnisse vorliegen:
Siliziumdioxid zu Gallium(III)oxid = 10 - 200
Hydroxylionen zu Siliziumdioxid = 0,05 - 0,5
Tetrapropylammoniumhydroxid zu Siliziumdioxid = 0 - 0,5
Wasser zu Siliziumdioxid = 20 - 100.

4. Verfahren nach Anspruch 1, 2 oder 3, dadurch gekennzeichnet, daß als quarternäre Ammoniumverbindung ein Tetrapropylammoniumsalz, wie z. B. Tetrapropylammoniumbromid oder Tetrapropylammoniumhydroxid, verwendet wird.

5. Verfahren nach einem der vorhergehenden Ansprüche, dadurch gekennzeichnet, daß das Keimbildungsgel bei atmosphärischem Druck im Bereich von 15 bis 100 °C über zwei Stunden bis 100 Tage gealtert wird.

6. Verfahren nach einem der vorhergehenden Ansprüche, dadurch gekennzeichnet, daß das Keimbildungsgel bei atmosphärischem Druck zwischen 20 °C und 90 °C über einen Zeitraum zwischen 2 und 7 Tagen gealtert wird.

7. Verfahren nach einem der vorhergehenden Ansprüche, dadurch gekennzeichnet, daß man das gealterte Keimbildungsgel Reaktionsansätzen mit folgender molaren Zusammensetzung beimischt:
SiO₂/Ga₂O₃ ≧ 5
OH⁻/SiO₂ = 0,05 - 0,5
quarternäre Ammoniumverbindung/SiO₂ = 0,0 - 0,5
H₂O/SiO₂ = 20 - 100
Me/SiO₂ = 0,3 - 3,0
wobei Me ein Alkali- oder Erdalkalimetall darstellt.

8. Verfahren nach einem der vorhergehenden Ansprüche, dadurch gekennzeichnet, daß das gealterte Keimbildungsgel in einer Menge von 2 bis 40 Gew.-% bezogen auf den Reaktionsansatz zugegeben wird.

9. Verfahren nach einem der vorhergehenden Ansprüche, dadurch gekennzeichnet, daß das gealterte Keimbildungsgel in einer Menge zwischen 5 und 37 Gew.-% bezogen auf den Reaktionsansatz zugegeben wird.

10. Verfahren nach einem der vorhergehenden Ansprüche, dadurch gekennzeichnet, daß die hydrothermale Kristallisation bei Temperaturen zwischen 40 und 300 °C durchgeführt wird.

11. Verfahren nach einem der vorhergehenden Ansprüche, dadurch gekennzeichnet, daß die hydrothermale Kristallisation bei Temperaturen zwischen 100 und 200 °C durchgeführt wird.

12. Verfahren nach einem der vorhergehenden Ansprüche, dadurch gekennzeichnet, daß man dem Reaktionsansatz außer dem Keimbildungsgel ein Fluorid, wie z. B. Natrium- oder Ammoniumfluorid, zugibt, wobei das Molverhältnis F⁻/SiO₂ zwischen 0,4 und 1,5 eingestellt wird.

13. Verfahren nach einem der vorhergehenden Ansprüche, dadurch gekennzeichnet, daß das kristalline, zeolithanaloge Gallosilikat ein Si/Ga-Atomverhältnis zwischen 5 und 100 aufweist.

14. Verfahren nach einem der vorhergehenden Anspürche, dadurch gekennzeichnet, daß die Reaktionszeit bei der hydrothermalen Kristallisation zwischen 1 h und 72 h beträgt.

15. Verfahren nach einem der vorhergehenden Ansprüche, dadurch gekennzeichnet, daß die Reaktionszeit bei der hydrothermalen Kristallisation zwischen 24 h und 48 h beträgt.

16. Verfahren nach einem der vorhergehenden Ansprüche, dadurch gekennzeichnet, daß die alkalische Lösung des Reaktionsansatzes eine Si-Verbindung, ausgewählt aus der Gruppe von Siliziumdioxid, Siliziumhydroxid und Alkalisilikaten oder deren Mischungen, enthält.

17. Verfahren nach einem der vorhergehenden Ansprüche, dadurch gekennzeichnet, daß die alkalische Lösung des Reaktionsansatzes eine Ga-Verbindung, ausgewählt aus der Gruppe von Ga(III)-oxid, Galliumhydroxid, Galliumnitrat, Galliumsulfat und Alkaligallaten oder deren Mischungen, enthält.

## Claims

1. A process of producing crystalline, zeolite-analogous gallosilicates with a pentasil structure with a Si/Ga atom ratio of ≧ 5 by hydrothermal crystallisation from a reaction mixture which, in an aqueous, alkaline medium, contains SiO₂ and Ga₂O₃ and, respectively, their hydratised derivates or alkali silicates and alkali gallates and, if applicable, quarternary ammonium compounds,
characterised in
that a crystallisation accelerating agent in the form of an aged, but still X-amorphous gallosilicate nucleation gel with a Si/Ga atom ratio ≧ 5 is added to the reaction mixture.

2. A process according to claim 1,
characterised in
that an aged nucleation gel of the following composition (on a molar basis) is used:
SiO₂/GaO₂ ≧ 5
OH⁻/SiO₂ = 0.01 to 1.0
quarternary ammonium compound/SiO₂ = 0.0 to 2.0
H₂O/SiO₂ = 10 - 1000.

3. A process according to any one of the preceding claims, characterised in
that the molar ratios in the nucleation gel are as follows: silicon dioxide to gallium(III) oxide = 10 - 200
hydroxil ions to silicon dioxide = 0.05 - 0.5
tetrapropyl ammonium hydroxide to silicon dioxide = 0 - 0.5
water to silicon dioxide = 20 - 100.

4. A process according to claims 1, 2 or 3,
characterised in
that the quarternary ammonium compound is used in the form of a tetrapropyl ammonium salt such as tetrapropyl ammonium bromide or tetrapropyl ammonium hydroxide.

5. A process according to any one of the preceding claims, characterised in
that the nucleation gel is aged at an atmospheric pressure ranging between 15 °C and 100 °C over a period of two hours to 100 days.

6. A process according to any one of the preceding claims, characterised in
that the nucleation gel is aged at an atmospheric pressure ranging between 20 °C and 90 °C over a period of 2 to 7 days.

7. A process according to any one of the preceding claims, characterised in
that the aged nucleation gel is mixed with reaction mixtures of the following molar composition:
SiO₂/Ga₂O₃ ≧ 5
OH⁻/SiO₂ = 0.05 - 0.5
quarternary ammonium compound/SiO₂ = 0.0 - 0.5
H₂O/SiO₂ = 20 - 100
Me/SiO₂ = 0.3 - 3.0,
With Me constituting an alkaline metal or alkaline-earth metal.

8. A process according to any one of the preceding claims, characterised in
that the aged nucleation gel is added in a quantity of 2 to 40 percent by weight with reference to the reaction mixture.

9. A process according to any one of the preceding claims, characterised in
that the aged nucleation gel is added in a quantity of 5 to 37 percent by weight with reference to the reaction mixture.

10. A process according to any one of the preceding claims,
characterised in
that hydrothermal crystallisation takes place at temperatures ranging between 40 and 300 °C.

11. A process according to any one of the preceding claims,
characterised in
that hydrothermal crystallisation takes place at temperatures ranging between 100 and 200 °C.

12. A process according to any one of the preceding claims,
characterised in
that in addition to the nucleation gel, a fluoride such as sodium or ammonium fluoride is added to the reaction mixture, with the molar ratio F⁻/SiO₂ being set between 0.4 and 1.5.

13. A process according to any one of the preceding claims,
characterised in
that the crystalline, zeolite-analagous gallosilicate comprises a Si/Ga atom ratio between 5 and 100.

14. A process according to any one of the preceding claims,
characterised in
that the reaction time during hydrothermal crystallisation ranges between 1 h and 72 h.

15. A process according to any one of the preceding claims,
characterised in
that the reaction time during hydrothermal crystallisation ranges between 24 h and 48 h.

16. A process according to any one of the preceding claims,
characterised in
that the alkaline solution of the reaction mixture contains a Si-compound selected from the group of silicon dioxide, silicon hydroxide and alkaline silicates or their mixtures.

17. A process according to any one of the preceding claims,
characterised in
that the alkaline solution of the reaction mixture contains a Ga compound selected from the group of Ga(III) oxide, gallium hydroxide, gallium nitrate, gallium sulphate and alkali gallates or their mixtures.

## Revendications

1. Procédé de préparation de gallosilicates cristallins, analogue à des zéolites, ayant une structure de pentasil et présentant un rapport atomique Si/GA ≧ 5, par cristallisation hydrothermale d'une charge de réaction qui contient dans un milieu alcalin aqueux SiO₂ et Ga₂O₃ ou leurs dérivés hydratés ou les silicates alcalins et des gallates alcalins et éventuellement des composés d'ammonium quaternaire,
procédé caractérisé en ce qu'on ajoute à la charge de réaction, à titre d'accélérateur de la cristallisation, un gel formateur de germes de gallosilicate , vieilli mais encore amorphe quand on l'examine aux rayons X, et qui présente un rapport atomique Si/Ga ≧ 5.

2. Procédé selon la revendication 1, caractérisé en ce qu'on utilise un gel vieilli, formateur de germes, ayant pour composition (sur base molaire) :
SiO₂/GaO₂ ≧ 5
OH⁻/SiO₂ = 0,01 à 1,0
Composé d'ammonium quaternaire/SiO₂ = 0,0 - 2,0
H₂O/SiO₂ = 10 - 1000.

3. Procédé selon l'une des revendications précédentes, caractérisé en ce que le gel formateur de germes présente les rapports molaires suivants :
Dioxyde de silicium à oxyde de gallium (III) = 10 - 200
Ion hydroxyle à dioxyde de silicium = 0,05 - 0,5
Hydroxyde de tétrapropylammonium à dioxyde de silicium = 0-0,5
Eau à dioxyde de silicium = 20 - 100.

4. Procédé selon la revendication 1, 2, ou 3, caractérisé en ce qu'on utilise comme composé d'ammonium quaternaire un sel de tétrapropylammonium comme, par exemple, le bromure de tétrapropylammonium ou l'hydroxyde de tétrapropylammonium.

5. Procédé selon l'une des revendications précédentes, caractérisé en ce qu'on soumet le gel, formateur de germes, à un vieillissement à la pression atmosphérique, dans l'intervalle de température allant de 15 à 100°C, en un intervalle de temps compris entre deux heures et 100 jours.

6. Procédé selon l'une des revendications précédentes, caractérisé en ce qu'on soumet le gel, formateur de germes, à vieillissement sous la pression atmosphérique, entre 20°C et 90°C en un intervalle de temps compris entre 2 et 7 jours.

7. Procédé selon l'une des revendications précédentes, caractérisé en ce qu'on incorpore le gel, formateur de germes et vieilli, à des charges de réaction ayant la composition molaire suivante :
SiO₂/Ga₂O₃ ≧ 5
OH⁻/SiO₂ = 0,05 - 0,5
Composé d'ammonium quaternaire/SiO₂ = 0,0 - 0,5
H₂O/SiO₂ = 20 - 100
Me/SiO₂ = 0,3 - 3,0
le symbole Me représentant un métal alcalin ou alcalino terreux.

8. Procédé selon l'une des revendications précédentes, caractérisé en ce qu'on ajoute le gel, formateur de germes et vieilli, en une quantité de 2 à 40 % en poids, par rapport à la charge de réaction.

9. Procédé selon l'une des revendications précédentes, caractérisé en ce qu'on ajoute le gel, formateur de germes et vieilli, en une quantité comprise entre 5 et 37% en poids, par rapport à la charge de réaction.

10. Procédé selon l'une des revendications précédentes, caractérisé en ce qu'on effectue la cristallisation hydrothermale à des températures comprises entre 40 et 300°C.

11. Procédé selon l'une des revendications précédentes, caractérisé en ce qu'on conduit la cristallisation hydrothermale à des températures comprises entre 100 et 200°C.

12. Procédé selon l'une des revendications précédentes, caractérisé en ce qu'on ajoute à la charge de réaction, en plus du gel formateur de germes, un fluorure comme par exemple le fluorure de sodium ou d'ammonium, en ajustant le rapport molaire F⁻/SiO₂ à une valeur comprise entre 0,4 et 1,5.

13. Procédé selon l'une des revendications précédentes, caractérisé en ce que le gallosilicate cristallin, analogue à de la zéolite, présence un rapport atomique Si/Ga compris entre 5 et 100.

14. Procédé selon l'une des revendications précédentes, caractérisé en ce que le temps de réaction de la cristallisation hydrothermale se situe entre 1 heure et 72 heures.

15. Procédé selon l'une des revendications précédentes, caractérisé en ce que le temps de réaction se situe pour la cristallisation hydrothermale entre 24 heures et 48 heures.

16. Procédé selon l'une des revendications précédentes, caractérisé en ce que la solution alcaline de la charge de réaction contient un composé de Si, choisi dans l'ensemble formé par le dioxyde de silicium, l'hydroxyde de silicium et des silicates alcalins ou leurs mélanges.

17. Procédé selon l'une des revendications précédentes, caractérisé en ce que la solution alcaline de la charge de réaction contient un composé de Ga, choisi dans l'ensemble formé par l'oxyde de Ga (III), de l'hydroxyde de gallium, du nitrate de gallium, du sulfate de gallium et des gallates alcalins ou leurs mélanges.
